(19) Europäisches Patentamt
European Patent Office
Office européen des brevets

(11) Numéro de publication: **0 000 453**
**B1**

(12) **FASCICULE DE BREVET EUROPEEN**

(21) Numéro de dépôt: **78400029.1**

(22) Date de dépôt: **21.06.78**

(51) Int. Cl.³: **C 07 D 495/04,**
C 07 D 333/16,//
C 07 C 149/16,
(C 07 D 495/04, 333/00,
221/00)

(54) **Procédé de préparation de dérivés de la thiénopyridine**

(30) Priorité: **12.07.77 FR 7721517**

(43) Date de publication de la demande:
**24.01.79 Bulletin 79 02**

(45) Mention de la délivrance du brevet:
**23.07.80 Bulletin 80 15**

(84) Etats contractants désignés:
**DE NL SE**

(56) Documents cités:
**78 400 029.1**
**Néant**

(73) Titulaire: **PARCOR**
**40, avenue Georges V**
**F-75008 Paris**
**(FR)**

(72) Inventeurs: **Braye, Emile**
**"Puntis"**
**F-31190 Lagardelle**
**Leze**
**FRANCE**
**Bousquet, André**
**2, Rue Léon Blum**
**F-31120 Portet**
**Garonne**
**(FR)**

(74) Mandataire: **Lavoix, Jean**
**c/o Cabinet Lavoix 2,**
**Place D'Estienne D'Orves**
**F-75441 Paris Cedex 09**
**(FR)**

EP 0 000 453 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Procédé de préparation de dérivés de la thiénopyridine

Procédé de préparation de dérivés de la thiénopyridine.—

La présente invention est relative à un procédé de préparation de dérivés de la thiénopyridine.

On a décrit dans la demande française 75 03 968 et son premier certificat d'addition 75 23 786 un procédé de préparation de thiénopyridine par réaction d'un dérivé de β-thiényl-2-éthylamine, éventuellement substitué, sur le formaldéhyde en présence d'un acide. Cette réaction est, de préférence, réalisée en deux étapes, tout d'abord par réaction de la β-thiényléthylamine sur le formaldéhyde puis cyclisation du produit obtenu en milieu anhydre par un acide anhydre. Cependant, le taux de conversion du produit de départ n'est que de l'ordre de 60 à 65%, ce qui oblige à un recyclage du produit de départ n'ayant pas réagi.

De plus ce procédé ne permet pas de préparer des dérivés de la thiénopyridine substitués en position 4 à l'aide de la réaction de Pictet Spengler par action d'un aldéhyde sur un dérivé de la thiényléthylamine.

On connaît également un procédé de préparation de 1,2,3,4-tétrahydroisoquinoléines, à partir de dérivés N-benzoylés de bêta-arylamine et de chlorométhylméthyléther, décrit dans Chemical Communications, 1969, 1283—4. Cependant il convient de remarquer que la cyclisation a lieu sur un noyau benzénique fortement activé et que le centre réactionnel est un amide.

La présente invention a pour but de remédier à ces inconvénients en fournissant un procédé permettant à la fois d'accéder aux dérivés substitués en position 4, et d'obtenir des rendements et taux de conversion nettement plus élevés.

Elle a ainsi pour objet un procédé de préparation de composés de formule générale

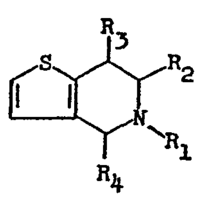

(I)

dans laquelle $R_1$ représente un atome d'hydrogène, un radical alcoyle, aryle ou aralcoyle éventuellement substitué, $R_2$ et $R_3$ sont identiques ou différents et représentent un atome d'hydrogène, un radical alcoyle inférieur, aryle ou hétérocyclique et $R_4$ représente un atome d'hydrogène, un radical alcoyle, un radical cycloalcoyle, alcoxy carbonyle, carboxy, aryle ou hétérocyclique, caractérisé en ce qu'on fait réagir un composé de formule

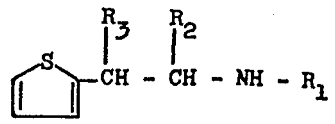

(II)

dans laquelle $R_1$, $R_2$ et $R_3$ sont tels que définis cil-dessus, avec un composé de formule

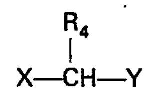

(III)

dans laquelle $R_4$ est tel que défini ci-dessus; X est un atome d'halogène, un groupe alcoxy éventuellement substitué, thioalcoyle éventuellement substitué ou amino éventuellement substitué; Y représente un groupe alcoxy éventuellement substitué, thioalcoyle éventuellement substitué, amino éventuellement substitué ou un groupe de formule

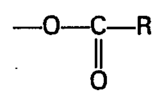

dans laquelle R est un radical alcoyle inférieur ou aryle, ou les deux groupes X et Y forment avec l'atome de carbone sur lequel ils sont fixés un noyau hétérocyclique à six chaînons hexahydro-S-triazinique, trioxannique ou trithianique, dans un solvant inerte, à une températuré comprise entre 0 et 150°C et dans un milieu anhydre, et éventuellement, on libère la base libre.

Les composés de formule III qui sont utilisés dans le procédé de l'invention peuvent être illustrés suivant la nature de X et Y par les composés suivants.

a) — un halogénométhyl éther du type

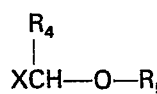

ou

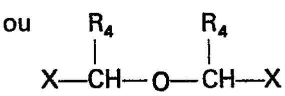

dans lequel X est un atome d'halogène tel que Cl ou Br et $R_5$ est un radical alcoyle inférieur ou aryle.

b) — un halogénométhyl thio éther du type

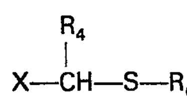

ou

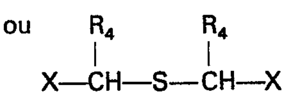

dans lequel X est un atome d'halogène tel que Cl ou Br et $R_6$ est un radical alcoyle inférieur ou aryle.

c) — un halogénométhyl ester du type

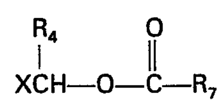

dans lequel X est un atome d'halogène tel que Cl ou Br et $R_7$ est un radical alcoyle inférieur ou aryle.

d)—une S -hexahydro-S-triazine de formule

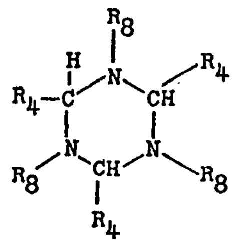

ou un dérivé aminé de formule

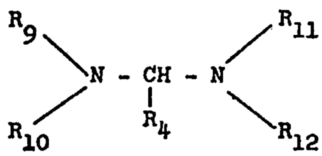

dans lesquelles $R_8$, $R_9$, $R_{10}$, $R_{11}$, $R_{12}$ sont des radicaux alcoyles inférieurs qui forment éventuellement entre eux un pont azoté ou des radicaux aryles, identiques ou différents.

e) — un trioxanne de formule

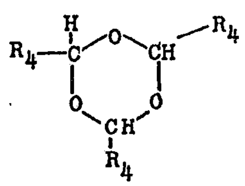

ou un dérivé de polyoxyméthylène de formule

$$R_{13}-(O-CH_2)_n OR_{14}$$

dans lesquelles $R_{13}$ et $R_{14}$ sont des atomes hydrogènes ou des radicaux alcoyles inférieurs ou aryles, identiques ou différents, et $n \geqslant 1$.

f) — un trithiane de formule

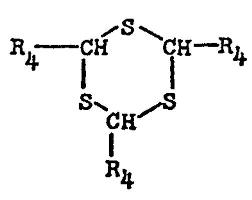

ou un dérivé de polythiométhylène de formule

$$R_{15}-(SCH_2)-_n SR_{16}$$

dans lesquelles $R_{15}$ et $R_{16}$ sont des radicaux alcoyles inférieurs ou aryles, identiques ou différents et $n \geqslant 1$.

La réaction entre le composé de formule II et le composé de formule III est réalisée dans un solvant inerte à une température comprise entre 0°C et 150°C, mais de préférence entre la température ambiante et le point d'ébullition du composé le plus volatil qui est en général un des solvants utilisés.

Cette réaction passe vraisemblablement par la formation d'un composé intermédiaire qui n'est pas isolé, mais cyclisé in situ, ainsi que cela sera exposé ci-après.

Or la cyclisation de ce composé intermédiaire nécessite la présence d'un acide qui est soit engendré dans le milieu par la réaction de formation de l'intermédiaire, soit est ajouté lorsque cette réaction de formation de l'intermédiaire n'en fournit pas. La formation d'acide in situ dépend de la nature du composé de formule III, ainsi pour les composés de formule III des types a, b, c, on observe la formation d'un tel acide de formule XH qui sert d'agent de cyclisation, alors que pour les composés de formule III des types d, e, et f, il ne se forme pas d'acide.

Dans ce dernier cas, on utilise un solvant de réaction qui contient un acide qui peut être par exemple:

un acide minéral anhydre, tel que les acides chlorohydrique, sulfurique, bromhydrique, phosphorique, un acide organique, carboxylique, tel que les acides oxalique, acétique, monochloracétique ou sulfonique, tel que les acides méthanesulfonique, benzènesulfonique.

Dans les cas où l'acide est engendré dans le milieu on préfère ajouter le composé de formule III dans la solution du composé de formule II, bien que l'inverse soit également possible. Dans les autres cas, on préfère ajouter un mélange des composés de formules II et III au solvant réactionnel contenant l'acide de cyclisation.

La réaction est le plus souvent rapide, mais il peut être parfois avantageux de chauffer en fin de réaction pour l'accélérer.

On peut opérer à la pression atmosphérique ou sous une pression supérieure, mais la pression atmosphérique est en général suffisante.

On réalise la réaction dans un solvant inerte vis à vis des réactifs, notamment des composés de formule III. Ce solvant doit être anhydre car l'eau décompose les composés de formule III. On utilise de préférence un solvant aprotique qui peut être de nature polaire, comme le diméthylformamide, le diméthylsulfoxyde, l'héxaméthyl phosphorotriamide ou un autre solvant, tel que le benzène, le toluène, un solvant chloré, tel qu'un hydrocarbure chloré ou des éthers légers.

Il est avantageux de réaliser la réaction dans un solvant dans lequel l'halogénure du composé de formule I est peu ou pas soluble car, en effet, on peut alors isoler le sel du composé de formule 1, à la fin de la réaction, par filtration du précipité formé. Ce mode opératoire permet, outre une commodité de mise en oeuvre, d'obtenir d'excellents rendements.

Les composés de formule II et III sont mis à réagir en quantités stoechiométriques, ou éventeuellement avec un excès molaire du composé de formule III allant jusqu'à 50% environ.

Bien que ne voulant pas être lié par un mécanisme réactionnel, la Demanderesse pense devoir indiquer que la réaction se passe en deux stades donnés dans le schéma réactionnel ci-après, ces deux stades n'étant pas distincts en pratique.

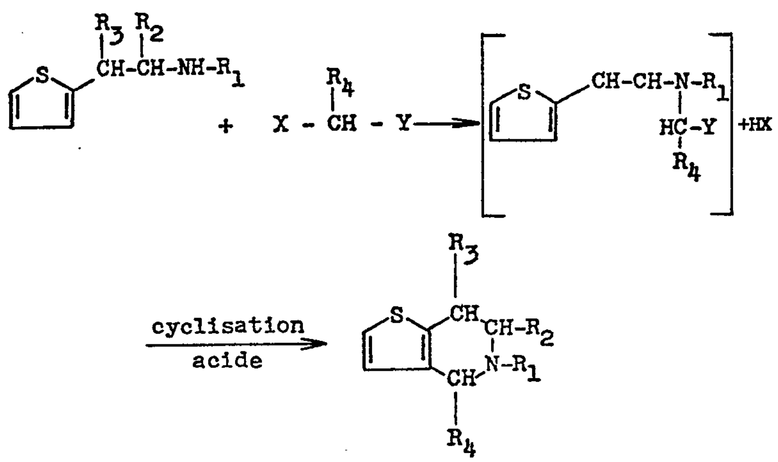


La cyclisation se fait donc avec formation d'un alcool, d'un mercaptan, d'une amine ou de l'eau.

Cette cyclisation est obtenue avec de très bons rendements et taux de conversion du produit de départ. Ainsi, dans les cyclisations où le réactif est le chlorométhyl méthyl éther, ou chlorométhyl méthylthioéther, le taux de conversion du composé de formula II est voisin de 100% et les rendements de l'ordre de 90 à 95%.

Par ailleurs, grâce au procédé de l'invention, on peut obtenir des dérivés de thiénopyridine dans lesquels le groupe $R_4$ est un groupe aryle tel que le groupe phényle, un groupe hétérocyclique tel que le groupe thiényl-2, un radical aliphatique ou cycloaliphatique ou un groupe fonctionnel tel que alcoxycarbonyle, carboxy.

La préparation des réactifs de départ de formule III est réalisée facilement par des procédés connus dans la littérature et qui n'entrent pas dans le cadre de l'invention. Certains des composés de formule III sont instables et il est alors nécessaire de les préparer juste avant leur emploi dans le procédé de l'invention où on les utilise sans purification.

Les exemples non limitatifs suivants sont donnés à titre d'illustration du procédé de l'invention.

Exemple 1

*Préparation de chlorhydrate de (chloro-2 benzyl) - 5 tétrahydro 4,5,6,7 thiéno [3,2-c] pyridine*

A une solution de 50,8 g (0,2 M) de N - (chloro-2 benzyl) (thiényl-2) - 2 éthylamine dans 70 cm$^3$ de diméthylformamide, chauffée à 60°C, on ajoute en 7 minutes 22,7 g (0,24 M) de chlorométhylméthyléther. La température du milieu réactionnel est maintenue à 60°C pendant toute la phase d'addition par refroidissement à l'eau. Trente minutes après la fin de l'addition du chlorométhylméthyléther le milieu est refroidi à 20°C. Le produit attendu, qui a précipité, est filtré et lavé avec deux fois 70 cm$^3$ d'acétone. On obtient 45,1 g de chlorhydrate de (chloro-2 benzyl) - 5 tétrahydro 4,5,6,7 thiéno [3,2-c] pyridine. (Rendement: 75%).

Le traitement des filtrats permet de récupérer encore 9 g du composé recherché (rendement 90% — P.F. = 190°C).

Exemple 2

*Préparation du chlorhydrate de (chloro-2 benzyl) - 5 éthoxy carbonyl- 4 tétrahydro 4,5,6,7 thiéno [3,2-c] pyridine.*

Dans un ballon tricol de 250 cm$^3$, on introduit 25,1 g (0,1 M) de N- (chloro-2 benzyl) (thiényl-2)-2 éthylamine en solution dans 30 cm$^3$ de diméthylformamide. On ajoute alors en 6 minutes 18,3 g (0,11 M) de chloro-2 éthoxy-2 acétate d'éthyle et on chauffe 4 heures à 80°C. Le produit attendu commence à précipiter. Après refroidissement du milieu, le produit est filtré et recristallisé trois fois dans 20 cm$^3$ d'acétone. On obtient 20,5 g de chlorhydrate de (chloro-2 benzyl) - 5 éthoxy-carbonyl-4 tétrahydro 4,5,6,7 thiéno [3,2-c] pyridine (rendement: 55%).

Le traitement des filtrats permet d'obtenir à nouveau 10 g de produit brut (P.F. : 156°C). Le produit brut est obtenu analytiquement pur par recristallisation dans un mélange éthanoléther isopropylique (rendement total : 81,8%).

Exemple 3

Préparation du chlorhydrate de (chloro-2

benzyl)-5 (thiényl 2) - 4 tétrahydro 4,5,6,7 thiéno [3,2-c] pyridine.

a) Préparation du (thiényl-2) chlorométhylméthyléther.

On charge dans un ballon, sous agitation, 112g (1 M) de (thiényl-2) carboxaldéhyde, 50 g (1,55 M) de méthanol, 125 $cm^3$ de chlorure de méthylène et 150 g de sulfate de sodium. On refroidit le milieu réactionnel à - 35°C et on fait passer jusqu'à saturation un courant d'acide chlorhydrique gazeux sec en empêchant la température de dépasser —20°C. Après arrêt du bullage de l'acide chlorhydrique, le milieu réactionnel est laissé à —20°C, sous agitation, pendant 2 heures. On évapore ensuite le chlorure de méthylène à —20°C pour obtenir le (thiényl-2) chlorméthyl-méthyléther brut.

b) Préparation de chlorhydrate de (chloro-2 benzyl) - 5 (thiényl-2) - 4 tétrahydro 4,5,6,7 thiéno [3,2-c] pyridine.

On fait réagir la N - (chloro-2 benzyl) (thiényl-2) - 2 éthylamine sur l'éther préparé sous (a) ci-dessus d'une manière analogue à celle décrite à l'exemple 1 pour obtenir le composé recherché (point de fusion de la base : 109°C).

Exemples 4 et 5

On prépare, d'une manière analogue à celle décrite dans l'exemple 1, les composés suivants:

le chlorhydrate de (chloro-2 benzyl) - 5 phényl - 4 tétrahydro 4,5,6,7 thiéno [3,2-c] pyridine
(P.F. de la base : 95°C),

le chlorhydrate de (chloro-2 benzyl) - 5 isopropyl - 4 tétrahydro 4,5,6,7 thiéno [3,2-c].
(P.F. de la base : 172°C),

respectivement à partir de N - (chloro-2 benzyl) (thiényl-2) - 2 éthylamine et des composés suivants:

—$\alpha$ chlorobenzyl-méthyléther,

— chloro-1 éthoxy-1 méthyl-2 propane

Exemple 6

*Préparation du chlorhydrate de tétrahydro 4,5,6,7 thiéno [3,2-c] pyridine*

A une solution de 12,7 g (0,1 M) de (thiényl-2) - 2-éthylamine dans 20 $cm^3$ de diméthylformamide, chauffée à 55°C, on ajoute en 10 minutes 8,05 g (0,1 M) de chlorométhyl-méthyléther dilué dans 10 $cm^3$ de diméthylformamide. Après l'addition du chlorométhylméthyléther le milieu est maintenu 2 heures à 70°C puis refroidi à la température ambiante. Le produit attendu, qui a précipité, est rincé à l'acétone. On obtient 5,5 g de chlorhydrate de tétrahydro 4,5,6,7 thiéno [3,2-c] pyridine (P.F. : 225°C). (Rendement : 31%).

Exemple 7

*Préparation du chlorhydrate de (chloro-2 benzyl) - 5 tétrahydro 4,5,6,7 thiéno [3,2-c] pyridine*

a) Préparation du chlorométhylméthylthioéther

Dans un ballon tricol d'une capacité de 1 litre, on charge 274 g (2,3 M) de chlorure de thionyle et 400 $cm^3$ de chlorure de méthylène. On chauffe au reflux (41°C) et on ajoute lentement 156 g de diméthylsulfoxyde. Tout au long de l'addition, on observe un important dégagement gazeux constitué de $SO_2$ et HCl. On doit maintenir un léger chauffage pour conserver le milieu au reflux. A la fin de l'addition du diméthylsulfoxyde, on fait passer un courant d'azote pour éliminer l'acide chlorhydrique dissous. Le milieu réactionnel (333 g) est utilisé tel quel dans l'opération de cyclisation décrite ci-après. Un dosage quantitatif par chromatographie en phase gazeuse donne les résultats suivants:

— chlorométhylméthylthioéther : 56,77%

— chlorure de méthylène : 33%

b) chlorhydrate de (chloro-2 benzyl) - 5 tétrahydro 4,5,6,7 thiéno [3,2-c] pyridine.

A 52 g (0,2 M) de N - (chloro-2 benzyl) (thiényl-2) - 2 éthylamine en solution dans 60 $cm^3$ de diméthylsulfoxyde et chauffés à 60°C, on ajoute en 30 minutes 51 g (0,3 M) de chlorométhylméthylthioéther brut préparé à l'étape (a) décrite ci-dessus. La température du milieu réactionnel monte alors progressivement et on la maintient entre 80 et 85°C pendant toute la phase d'addition par refroidissement à l'eau. A la fin de l'addition du chlorométhylméthylthioéther le milieu est refroidi à 6°C. Le produit attendu précipite facilement. Après filtration et lavage avec 2 x 70 $cm^3$ d'acétone, on obtient 44,1 g de chlorhydrate de (chloro-2 benzyl) - 5 tétrahydro 4,5,6,7 thiéno [3,2-c] pyridine (rendement 73,5%). Le traitement des filtrats permet de récupérer encore 10 g du composé recherché (rendement : 90% — P.F. = 190°C). Le produit brut ainsi obtenu contient de 1 à 2% d'impuretés et peut être rendu analytiquement pur par recristallisation dans l'éthanol.

Exemple 8

*Préparation de chlorhydrate de (chloro-2 benzyl)* - 5 tétrahydro 4,5,6,7 thiéno [3,2-c] pyridine

Le même composé qu'à l'exemple 1 est préparé comme suit.

On fait réagir, sous chauffage pendant 3 heures, 66,5 g (0,44 M) de pivalate de chlorométhyle sur une solution de 103 g (0,4 M) de N - (chloro-2 benzyl) (thienyl-2) - 2 éthylamnine dans 300 $cm^3$ de diméthylsulfoxyde, pour obtenir avec un rendement de 51% le composé indiqué dans le titre.

Exemple 9

*Préparation du chlorhydrate de (chloro-2 benzyl)* - 5 tétrahydro 4,5,6,7 thiéno [3,2-c] pyridine.

A 42 $cm^3$ de diméthylformamide dans lesquels on a dissous 0,25 mole d'acide chlorhydrique gazeux et que l'on a chauffé à 40°C, on ajoute en 2 minutes un mélange de 25,15 g (0,1 M) de N - (chloro-2 benzyl) (thiényl-2) - 2 éthylamine et de 15,3 g (0,033 M) de s. hexahydrotriazine de l'o.chlorobenzylamine. La réaction est exothermique et durant l'addition, on doit refroidir à l'eau pour maintenir la température du milieu réactionnel inférieure à 70°C. A la fin de l'addition on laisse 30 minutes sous agitation puis on refroidit. On filtre et lave 2 fois à l'acétone le produit attendu. On obtient 17,3 g de chlorhydrate (chloro-2 benzyl) - 5 tétrahydro 4,5,6,7 thiéno [3,2-c] pyridine. Le traitement des filtrats permet de récupérer 10 g supplémentaires du produit attendu (rendement global : 90%).

Exemples 10 à 12

*Préparation du chlorhydrate de (chloro-2 benzyl)* - 5 tétrahydro 4,5,6,7 thiéno [3,2-c] pyridine

Suivant le même mode opératoire que décrit à l'exemple 9, on obtient le composé indiqué dans le titre en remplaçant la s.hexahydrotriazine de l'o.chlorbenzylamine par :

— la s. hexahydrotriazine de la n.butylamine (rendement ≃ 90%)

— l'urotropine (rendement : 60%)

—le paraformaldéhyde (rendement : 83%)

Exemple 13

*Préparation du chlorhydrate de tétrahydro 4,5,6,7* thiéno [3,2-c] pyridine

A 73 $cm^3$ de diméthylformamide chauffés à 45°C, dans lesquels on a dissous 0,45 mole d'acide chlorhydrique gazeux, on ajoute en 25 minutes un mélange de 17 g (0,2 M) de s.hexahydrotriazine de la n.butylamine et de 26 g de (thiényl-2) - 2 éthylamine. La température du milieu est maintenue à 45°C pendant l'addition à l'aide d'un bain d'eau froide. En fin d'addition, le produit attendu précipite. Par filtration, on obtient 22,16 g de chlorhydrate de tétrahydro 4,5,6,7 thieno [3,2-c] pyridine (rendement 65%).

Exemple 14

*Préparation du chlorhydrate de (chloro-2 benzyl)* - 5 tétrahydro 4,5,6,7 thiéno [3,2-c] pyridine

A 2,25 g (0,075 M) de paraformaldéhyde en suspension dans 20 $cm^3$ de diméthylformamide, on ajoute 9,61 g (0,1 M) d'acide méthanesulfonique. La température du milieu étant à 72°C, on ajoute en 2 minutes 13 g (0,05 M) de N-(chloro-2 benzyl) (thiényl-2) - 2 éthylamine dissous dans 5 $cm^3$ de diméthylformamide. La température du milieu atteint 90°C. Le milieu est alors refroidi à 20°C et versé sur 50 $cm^3$ de soude 4 N. On extrait alors avec 30 $cm^3$, puis 20 $cm^3$ de chlorure de méthylène. Les phases organiques sont rassemblées, séchées sur sulfate de sodium et évaporées. On obtient une huile qui est reprise par 30 $cm^3$ d'éthanol dans lequel on a dissous 0,15 mole d'acide chlorhydrique gazeux. Après évaporation partielle de l'éthanol, le produit attendu précipite. On filtre, lave à l'acétone et sèche. On obtient 11,35 g de chlorhydrate de N-(chloro-2 benzyl) - 5 tétrahydro 4,5,6,7 thiéno [3,2-c] pyridine (rendement 75,6%).

**Revendications**

1. Procédé de préparation de composés de formule générale

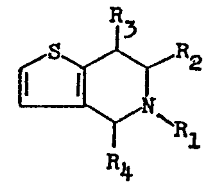

(I)

dans laquelle $R_1$ représente un atome d'hydrogène, un radical alcoyle, aryle ou aralcoyle, éventuellement substitué, $R_2$ et $R_3$ sont identiques ou différents et représentent un atome d'hydrogène, un radical alcoyle inférieur, aryle ou hétérocyclique et $R_4$ représente un atome d'hydrogène, un radical alcoyle, un radical cycloalcoyle, alcoxy carbonyle, carboxy, aryle ou hétérocyclique, caractérisé en ce qu'on fait réagir un composé de formule

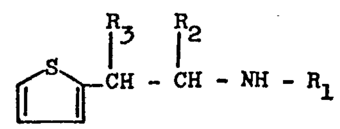

(II)

dans laquelle $R_1$, $R_2$ et $R_3$ sont tels que définis ci-dessus, avec un composé de formule

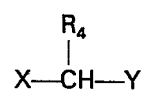

(III)

dans laquelle $R_4$ est tel que défini ci-dessus; X est un atome d'halogène, un groupe alcoxy éventuellement substitué, thioalcoyle éventuellement substitué ou amino éventuellement substitué; Y représente un groupe alcoxy éventuellement substitué, thioalcoyle éventuellement substitué, amino éventuellement substitué ou un groupe de formule

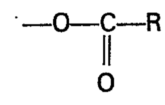

dans laquelle R est un radical alcoyle inférieur ou aryle; ou les deux groupes X et Y forment avec l'atome de carbone sur lequel ils sont fixés un noyau hétérocyclique à six chaînons hexahydro-S-triazinique, trioxannique ou trithianique, dans un solvant inerte, additionné,

lorsque X n'est pas un atome d'halogène, d'un acide minéral ou organique du type carboxylique ou sulfonique, à une température comprise entre 0 et 150°C et dans un milieu anhydre, et éventuellement on libère la base libre.

2. Procédé selon la revendication 1, caractérisé en ce que la réaction est réalisée entre la température ambiante et le point d'ebullition du composé le plus volatil.

3. Procédé selon la revendication 1, caractérisé en ce que le solvant inerte est un solvant aprotique polaire tel que le diméthylformamide, le diméthylsulfoxyde, l'hexaméthylphosphorotriamide, ou le benzène ou un solvant chloré tel qu'un hydrocarbure chloré ou un éther léger.

4. Procédé selon la revendication 1, caractérisé en ce que l'acide est l'acide chlorhydrique, ou méthane sulfonique.

5. Procédé selon la revendication 3, caractérisé en ce que le solvant est choisi de façon à être un non solvant de l'halogénure du composé de formule I.

6. Procédé selon la revendication 1, caractérisé en ce qu'on fait réagir le composé de formule III en une quantité comprise entre la quantité stoechiométrique et un excès molaire de 50%, par rapport au composé de formule II.

## Claims

1. Process for the preparation of compounds having the general formula:

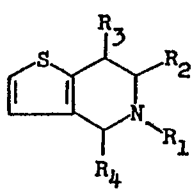

(I)

in which : $R_1$ represents a hydrogen atom, an optionally substituted alkyl, aryl or aralkyl radical; $R_2$ and $R_3$ are the same or different and represent independently a hydrogen atom, a lower alkyl, aryl or heterocyclic radical; and $R_4$ represents a hydrogen atom or an alkyl, cycloalkyl, alkoxy carbonyl, carboxy, aryl or heterocyclic radical, comprising reacting a compound having the formula :

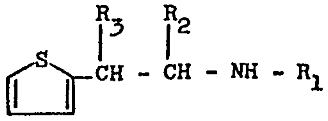

(II)

in which $R_1$, $R_2$ and $R_3$ are as defined above, with a compound having the formula :

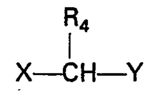

(III)

in which $R_4$ is as defined above; X is a halogen atom, or an optionally substituted alkoxy, thioalkyl or amino group; and Y represents an optionally substituted alkoxy, thioalkyl or amino group, or a group of the formula :

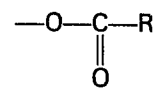

in which R is a lower alkyl or aryl radical, or both the groups X and Y, together with the carbon atom to which they are attached, form a 6-membered hexahydro-S-triazinic, trioxannic or trithianic heterocyclic nucleus, in an inert solvent to which is added an inorganic or organic acid or carboxylic or sulfonic type when X is other than a halogen atom, at a temperature between 0 and 150°C and in an anhydrous medium and, if desired, liberating the free base.

2. Process as claimed in claim 1, wherein the reaction is effected between room temperature and the boiling point of the more volatile compound.

3. Process as claimed in claim 1, wherein said inert solvent is a polar aprotic solvent such as dimethylformamide, dimethylsulfoxyde, hexamethylphosphorotriamide, or benzene or a chlorinated solvent such as chlorinated hydrocarbon or a light ether.

4. Process as claimed in claim 1, wherein said acid is hydrochloric acid or methane sulfonic acid.

5. Process as claimed in claim 3, wherein the solvent is selected so as to be a non-solvent for the halide of the compound of the formula (I).

6. Process as claimed in claim 1, wherein the compound of the formula (III) is reacted in an amount comprised between the stoichiometric amount and a molar excess of 50% with respect to the compound of the formula (II).

## Patentansprüche

1. Verfahren zur Herstellung von Thienopyridinderivaten der allgemeinen Formel

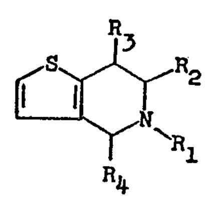

(I)

worin $R_1$ Wasserstoff, Alkyl, Aryl oder gegebenenfalls substituiertes Aralkyl bedeutet, $R_2$ und $R_3$ gleich oder verschieden sind und jeweils Wasserstoff, nied. Alkyl, Aryl oder eine hetero-

cyclische Gruppe darstellen und $R_4$ Wasserstoff, Alkyl, Cycloalkyl, Alkoxy-carbonyl, Carboxy, Aryl oder eine heterocyclische Gruppe ist, dadurch gekennzeichnet, daß man eine Verbindung de allgemeinen Formel

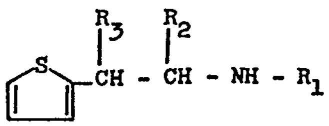

(II)

worin $R_1$, $R_2$ und $R_3$ die obige Bedeutung haben, mit einer Verbindung der allgemeinen Formel

$$X-\underset{}{\overset{R_4}{\overset{|}{C}H}}-Y \qquad (III)$$

worin $R_4$ die obige Bedeutung hat, X Halogen, gegebenenfalls substituiertes Alkoxy, gegebenenfalls substituiertes Thioalkyl oder gegebenenfalls substituiertes Amino bedeutet und Y gegebenenfalls substituiertes Alkoxy, gegebenenfalls substituiertes Thioalkyl, gegebenenfalls substituiertes Amino oder eine Gruppe der Formel

$$-O-\underset{\underset{O}{\|}}{C}-R$$

darstellt, wobei R nied. Alkyl oder Aryl ist, oder X und Y zusammen mit dem Kohlenstoffatom, an dem sie hängen, einen sechsgliedrigen heterocyclischen Hexahydro-S-triazin-, Trioxan- oder Trithianring bilden, in einem inerten Lösungsmittel das, wenn X ist nicht Halogen, eine Mineralsäure oder eine organische Carbon- oder Sulfosäure enthält, bei einer Temperatur von 0 bis 150°C und in wasserfreiem Medium umsetzt und gegebenenfalls die Base freisetzt.

2. Verfahren nach Anspruch 1, dadurch gekennzeichnet, daß man die Reaktion bei Umbebungstemperatur bis zum Siedepunkt der flüchtigeren Verbindung durchfürht.

3. Verfahren nach Anspruch 1, dadurch gekennzeichnet, daß man als inertes Lösungsmittel ein aprotisches polares Lösungsmittel, wie Dimethylformamid, Dimethylsulfoxyd, Hexamethylphosphortriamid oder Benzol, oder ein Chlorlösungsmittel, wie einen Chlorkohlenwasserstoff, oder einen niederen Äther einsetzt.

4. Verfahren nach Anspruch 1, dadurch gekennzeichnet, daß man als Säure Salzsäure oder Methansulfonsäure einsetzt.

5. Verfahren nach Anspruch 3, dadurch gekennzeichnet, daß man ein Lösungsmittel einsetzt, das das Halogenid der Verbindung der Formel (I) nicht löst.

6. Verfahren nach Anspruch 1, dadurch gekennzeichnet, daß Man die Verbindung der Formel (III) in stöchiometrischer Menge bis zu einem molaren überschß von 50%, bezogen auf die Verbindung der Formel (II), einsetzt.